(19) Europäisches Patentamt
European Patent Office
Office européen des brevets

(11) **EP 3 854 881 A1**

(12) **EUROPEAN PATENT APPLICATION**

(43) Date of publication:
**28.07.2021 Bulletin 2021/30**

(51) Int Cl.:
*C12Q 1/68* (2018.01)    *C12Q 1/6876* (2018.01)

(21) Application number: **20153236.3**

(22) Date of filing: **22.01.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BEIERSDORF AG**
**20245 Hamburg (DE)**

(72) Inventors:
• **HOLZSCHECK, Nicholas**
  **22549 Hamburg (DE)**
• **WINNEFELD, Marc**
  **22880 Wedel (DE)**
• **SCHLÄGER, Torsten**
  **22395 Hamburg (DE)**
• **GRÖNNIGER, Elke**
  **22303 Hamburg (DE)**

(74) Representative: **Haseltine Lake Kempner LLP**
**Bürkleinstrasse 10**
**80538 München (DE)**

(54) **CLASSIFYING SUBJECTS BASED ON THEIR BIOLOGICAL RESPONSE TO UV IRRADIATION**

(57)    The present invention relates to methods for classifying humans into Molecular Phototypes based on the biological response of their skin to UV irradiation. The method comprises: a) providing skin cells irradiated with UV light obtained from a plurality of human subjects; b) determining the methylation levels and/or expression levels of at least about 100 features in said skin cells, wherein the features are selected from CpG sites and RNAs; c) using the methylation data and/or expression data obtained in step b) to generate a subject similarity network; d) performing cluster analysis on the subject similarity network; and e) defining Molecular Phototypes based on the clusters in the subject similarity network. The invention also relates to methods for identifying biological pathways that are associated with the response to UV irradiation of skin in human subjects belonging to a Molecular Phototype determined by a method described herein. The method comprises: a) providing skin cells irradiated with UV light and control skin cells obtained from a plurality of human subjects belonging to a Molecular Phototype determined by a method described herein; b) determining the expression levels of at least one gene in said irradiated and control skin cells, wherein the at least one gene is annotated to a biological pathway; c) using a machine-learning model trained on the data obtained in step b) to identify whether said at least one gene can discriminate between irradiated and control skin cells; and d) determining the strength of the association between the biological pathway and the response to UV irradiation of skin in the human subjects belonging to the Molecular Phototype, wherein the strength of the association is based on the degree to which the machine-learning model can discriminate between irradiated and control skin cells. Also provided are computer programs relating to the methods.

Figure 1

## Description

### FIELD OF THE INVENTION

The present invention relates to methods for classifying humans into Molecular Phototypes based on the biological response of their skin to UV irradiation. In particular, methylation and/or RNA expression data are used to generate clustered subject similarity networks, which reveal discrete Molecular Phototypes. The present invention also relates to methods for identifying biological pathways that are associated with the response to UV irradiation of skin in human subjects belonging to a Molecular Phototype determined by a method defined herein.

### BACKGROUND OF THE INVENTION

It is known that exposure of the skin to radiation from the ultraviolet (UV) region of the light spectrum can have harmful effects on the skin, including permanent skin damage, discoloration, and premature aging, as well as DNA damage, which can lead to the development of skin cancer.

The biological response of the skin to UV irradiation can vary widely between individuals. Therefore, subtyping individuals according to their response to UV irradiation can be useful in biological research and medicine.

The Fitzpatrick phototyping scale classifies subjects as Fitzpatrick phototype I-VI based on their skin's complexion and propensity to tanning and burning in response to UV irradiation (Fitzpatrick (1975) Soleil et peau. J Med Esthet. (2):33-34; Eilers et al (2013) Accuracy of Self-report in Assessing Fitzpatrick Skin Phototypes I through VI. JAMA Dermatol. 149(11):1289-1294). However, since these variables are continuous and highly subjective, the Fitzpatrick classification system is poorly reproducible. Therefore, Fitzpatrick phototyping is generally only used when a very fast assessment is required.

So far, there is no objective, precise, and reproducible way to classify or subtype subjects based on the biological response of their skin to UV irradiation.

### SUMMARY OF THE INVENTION

The present invention is defined in the appended claims.

In accordance with a first aspect, there is provided a method for classifying humans into Molecular Phototypes based on the biological response of their skin to UV irradiation, the method comprising:

    a) providing skin cells irradiated with UV light obtained from a plurality of human subjects;
    b) determining the methylation levels and/or expression levels of at least about 100 features in said skin cells, wherein the features are selected from CpG sites and RNAs;
    c) using the methylation data and/or expression data obtained in step b) to generate a subject similarity network;
    d) performing cluster analysis on the subject similarity network; and
    e) defining Molecular Phototypes based on the clusters in the subject similarity network.

In accordance with a second aspect, there is provided computer program for classifying humans into Molecular Phototypes based on the biological response of their skin to UV irradiation, the computer program comprising instructions which, when the program is executed by a computer, cause the computer to carry out the following steps:

    a) inputting the methylation levels and/or expression levels of at least about 100 features in skin cells irradiated with UV light obtained from a plurality of human subjects, wherein the features are selected from CpG sites and RNAs;
    b) using the methylation data and/or expression data obtained in step a) to generate a subject similarity network;
    c) performing cluster analysis on the subject similarity network; and
    d) outputting definitions of Molecular Phototypes based on the clusters in the subject similarity network.

In a third aspect, there is provided a method for identifying biological pathways that are associated with the response to UV irradiation of skin in human subjects belonging to a Molecular Phototype determined by a method described herein, the method comprising:

    a) providing skin cells irradiated with UV light and control skin cells obtained from a plurality of human subjects belonging to a Molecular Phototype determined by a method described herein;
    b) determining the expression levels of at least one gene in said irradiated and control skin cells, wherein the at least one gene is annotated to a biological pathway;
    c) using a machine-learning model trained on the data obtained in step b) to identify whether said at least one gene can discriminate between irradiated and control skin cells; and
    d) determining the strength of the association between the biological pathway and the response to UV irradiation of skin in the human subjects belonging to the Molecular Phototype, wherein the strength of the association is based on the degree to which the machine-learning model can discriminate between irradiated and control skin cells.

In a fourth aspect of the invention, there is provided a computer program for identifying biological pathways that are associated with the response to UV irradiation of skin in human subjects belonging to a Molecular Phototype determined by a method described herein, the computer program comprising instructions which, when the program is executed by a computer, cause the computer to carry out the following steps:

a) inputting the expression levels of at least one gene obtained from skin cells irradiated with UV light and control skin cells obtained from a plurality of human subjects belonging to a Molecular Phototype determined by the method described herein, wherein the at least one gene is annotated to a biological pathway;

b) using a machine-learning model trained on the data obtained in step a) to identify whether said at least one gene can discriminate between irradiated and control skin cells;

c) determining the strength of the association between the biological pathway and the response to UV irradiation of skin in the human subjects belonging to the Molecular Phototype, wherein the strength of the association is based on the degree to which the machine-learning model can discriminate between irradiated and control skin cells; and

d) outputting a decision on whether the biological pathway is associated with the response to UV irradiation of skin in human subjects belonging to the Molecular Phototype.

Certain aspects and embodiments of the present invention may provide one or more of the following advantages:

- desired ability to objectively classify subjects according to their biological response to UV irradiation;
- desired ability to precisely classify subjects according to their biological response to UV irradiation;
- desired ability to reproducibly classify subjects according to their biological response to UV irradiation;
- desired ability to identify biological pathways associated with UV irradiation of skin in particular subjects.

The details, examples, and preferences provided in relation to any particular one or more of the stated aspects of the present invention apply equally to all aspects of the present invention. Any combination of embodiments, examples, and preferences described herein in all possible variations thereof is encompassed by the present invention unless otherwise indicated herein, or otherwise clearly contradicted by context.

## BRIEF DESCRIPTION OF THE DRAWINGS

The invention will further be illustrated by reference to the following figures:

Figure 1 provides a fused similarity network generated by integration of methylation and RNA expression data from irradiated skin samples of 32 subjects. The nodes are labelled according to the Molecular Phototypes identified through spectral clustering.
Figure 2 provides a heat-map showing the predictivity of the most characteristic pathways for each of the Molecular Phototypes identified in Figure 1.

It is understood that the following description and references to the figures concern exemplary embodiments of the present invention and shall not be limited on the scope of the claims.

## DETAILED DESCRIPTION

The present invention is based on the surprising finding that subjects cluster into discrete subtypes ("Molecular Phototypes") based on the molecular biological responses (methylation levels and RNA expression levels) of their skin cells to UV irradiation. This was surprising because the complexion of subjects' skin and their propensity to tanning and burning are continuous variables.

### Subjects

As used herein, "subjects" refers to human subjects.

As used herein, "a plurality of human subjects" may mean at least 3, 5, 10, 15, 20, 30, or 32 human subjects. In certain embodiments, "a plurality of human subjects" may mean at least 20 human subjects. In certain embodiments, "a plurality of human subjects" may mean at least 30 human subjects. The plurality of human subjects may be homogenous with regard to gender, age, and/or ethnicity.

Ideally, subjects had not taken anti-histamine, anti-inflammatory, anti-coagulant, immunosuppressive, or corticoid- or retinoid-containing drugs for at least two weeks prior to skin irradiation and/or sampling. Ideally, subjects had not used tanning or sun-protection products at least one week prior to skin irradiation and/or sampling. Ideally, subjects had not been sun bathing or visited a solarium at least four weeks prior to skin irradiation and/or sampling. Ideally, subjects did not have skin pigmentation disorders, in particular pigmentation disorders.

In certain embodiments, the subjects may be at least about 16, at least about 18, or at least about 30 years old. In certain embodiments, the subjects may be up to about 65 or up to about 80 years old. In certain embodiments, the subjects may be from about 18 years old to about 80 years old or from about 30 years old to about 65 years old.

In certain embodiments, subjects may be phototype I-VI on the Fitzpatrick scale (I meaning always burns, never tans; II meaning burns easily, then develops a light tan; III meaning burns moderately, then develops a light tan; IV meaning burns minimally to rarely, then develops a moderate tan; V meaning never burns, always develops a dark tan; VI meaning never burns, no noticeable change in appearance). In certain embodiments, subjects may be phototype I-IV on the Fitzpatrick scale. In certain embodiments, subjects may be Caucasian.

**UV irradiation and controls**

Methods described herein use test skin cells that have been irradiated with UV light and, in some embodiments, control skin cells that have not been irradiated with UV light.

Minimal erythema dose (MED) is the minimum dose of UV (measured in mJ/cm$^2$) which results in erythema (redness) and/or oedema (swelling) in a subject's skin 24 to 48 h after exposure to UV radiation. A subject's MED can be determined as previously described (International Organization for Standardization *DIN EN ISO 24444* (2010)).

As used herein, "ultraviolet radiation" or "UV radiation" refers to electromagnetic radiation with a wavelength of from about 100nm to about 400 nm, including UVC radiation (from about 100nm to about 280nm), UVB radiation (from about 280nm to about 315nm), and UVA radiation (from about 315nm to about 400nm). In certain embodiments, UV irradiation of the subjects' skin may have been performed using UV light having a wavelength of from about 100nm to about 400nm, or about 280nm to about 400nm. In certain embodiments, the UV irradiation may have been performed using UVA and UVB light. In certain embodiments, the UV irradiation may have been performed using a light source that simulates solar light, for example a SOL 500 full spectrum solar simulator (Honle UV Technology).

As used herein, test skin cells "irradiated with UV light" means skin cells intentionally irradiated with UV light, ideally artificial UV light, but does not include skin cells incidentally irradiated with UV light, especially sunlight.

In certain embodiments, the skin cells obtained from each subject may have been irradiated with an amount of UV light that achieved an MED of at least about 0.5, at least about 0.7, or at least about 0.9. In certain embodiments, the skin cells obtained from each subject may have been irradiated with an amount of UV light that achieved an MED of from about 0.5 to about 1.3, from about 0.7 to about 1.1, from about 0.8 to about 1.0, or about 0.9. In particular embodiments, the skin cells obtained from each subject may have been irradiated with an amount of UV light that achieved an MED of from about 0.8 to about 1.0. In certain embodiments, the skin cells obtained from each subject may have been irradiated with an amount of UV light that achieved an MED of about 0.9 (in other words, about 90% of the required minimal dose causing erythema in that subject).

In certain embodiments, the irradiation of each subject's skin may have been repeated (on the same area of skin). In other words, the irradiation may have been performed at least twice. Optionally, the irradiation may have been performed at least three times. Optionally, the irradiation may have been performed 2-4 times. In some specific embodiments, the irradiation may have been performed precisely three times.

In embodiments where irradiation may have been repeated, the irradiations may have been performed about 12 hours to about 48 hours apart, or about 12 hours to about 36 hours apart, about 20 to about 28 hours apart, or about 24 hours apart. In certain embodiments, the irradiations may have been performed about 12 to about 36 hours apart. In some specific embodiments, the irradiations may have been performed about 24 hours apart.

In one example, the irradiation may be performed at least twice, about 12 to about 36 hours apart. In another example, the irradiation may be performed at least three times, about 12 to about 36 hours apart. In another example, the irradiation may be performed at least three times, about 20 to about 28 hours apart. In another example, the irradiation may be performed at least three times, about 24 hours apart.

In certain embodiments, the skin cells may have been obtained from the subject within about 72 hours, about 48 hours, about 36 hours, or about 24 hours after the irradiation (if irradiation was repeated, after the final irradiation). In certain embodiments, the skin cells may have been obtained from the subject within about 12 to about 36 hours after the irradiation (the final irradiation, if irradiation was repeated). For example, the skin cells may have been obtained at about 24 hours after the irradiation (the final irradiation, if irradiation was repeated).

As used herein, control skin cells "not irradiated with UV light" means skin cells that have not been intentionally irradiated with UV light, especially not artificial UV light or UV light that caused skin discoloration (tanning or burning), for at least about 2 days, about 4 days, about 6 days, about one week, about two weeks, or about one month prior to sampling. However, control skin cells may include skin cells that have been incidentally irradiated with UV light, for example sunlight. Hence, in certain embodiments, control skin cells may have been obtained from a sun-protected area of the subject's body, for example the subjects' abdomens (e.g. lower backs) or upper limbs.

In certain embodiments, control skin cells may not have been irradiated with an amount of UV light that achieved an MED of more than about 0.1, 0.3, or 0.5 for at least about 2 days, about 4 days, about 6 days, about one week, about two weeks, or about one month prior to sampling. As an example, control skin cells may not have been irradiated with an amount of UV light that achieved an MED of more than about 0.5 for at least about one week prior to sampling. As another example, control skin cells may not have been irradiated with an amount of UV light that achieved an MED of more than about 0.3 for at least about two weeks prior to sampling. In other embodiments, control skin cells may not include skin cells that have been exposed to any UV light at all.

## Sampling of skin cells

As used herein, "a skin sample" or "skin cells" refers to a sample comprising skin cells as a majority, consisting essentially of skin cells, or consisting of skin cells. In certain embodiments at least 90%, at least 95%, at least 96%, at least 97%, at least 98%, or at least 99% of cells in the sample are skin cells.

In certain embodiments, the skin cells may have been/may be obtained by harvesting the entire skin sample required from the individual. Harvesting a sample from the individual may be carried out using suction blistering, punch biopsy, shave biopsy or during any surgical procedure such as plastic surgery, lifting, grafting, or the like. In certain embodiments, the sample may have been/may be obtained by suction blistering.

In certain embodiments, the skin cells may have been/may be obtained by culturing the skin cells using an *in vitro* method. Skin cells may have been/may be cultured from a small sample of skin cells harvested from an individual. The harvested human skin cells may have been/ may be grown *in vitro* in a vessel such as a petri dish in a medium or substrate that supplies essential nutrients.

Skin cells used in the methods described herein have been/are obtained from the epidermis and/or dermis, or derive from the epidermis and/or dermis. Hence, the skin cells may comprise epidermal cells and/or dermal cells as a majority of the cells in the sample. The skin cells may consist essentially of epidermal cells and/or dermal cells. The skin cells may consist of epidermal cells and/or dermal cells. In certain embodiments, the skin cells may comprise, consist, or consist essentially of epidermal cells. The skin cells may comprise a mixture of harvested cells and cultured cells.

In certain embodiments, the skin cells may have been obtained from a generally sun-protected area of the subject's body, for example on the subjects' lower backs. In certain embodiments, the skin cells have been obtained from areas of skin that do not have skin disorders (in particular pigmentation disorders), skin damage (e.g. moles), scars, or tattoos.

## CpG methylation analysis

As used herein, a "CpG site" (also referred to as a CpG dinucleotide) is a cytosine nucleotide immediately followed by a guanine nucleotide in the 5' to 3' direction within a DNA molecule. CpG sites may be in coding or non-coding regions of the genome. CpG sites may be in CpG islands, which are regions having a high density of CpG sites.

The cytosine in a CpG site can be methylated by DNA methyltransferases to become 5-methylcytosine. It is known that methylation of CpG sites within a gene can influence the transcriptional regulation and thus expression of the gene (epigenetic regulation).

In certain embodiments of the present invention, the methylation level of CpG sites may be determined, for example, by methylation specific PCR, sequence analysis of bisulfite treated DNA, CHIP-sequencing (e.g. the Infinium MethylationEPIC BeadChip Kit, the HumanMethylation450 BeadChip, or the Infinium HumanMethylation27 BeadChip, all by Illumina), molecular inversion probe assay, Methyl-CAP-sequencing, Next-Generation-sequencing, COBRA-Assay, methylation specific restriction patterns, or MassARRAY assay. In preferred embodiments the methylation level of CpG sites may be determined using the Infinium MethylationEPIC BeadChip array (Illumina). Alternatively, the HumanMethylation450 BeadChip (Illumina) or the Infinium HumanMethylation27 BeadChip (Illumina) may be used.

The Infinium MethylationEPIC BeadChip array (Illumina) can determine the methylation level of over 850,000 CpG sites, which are defined in the "Infinium MethylationEPIC v1.0 B4 Manifest File" available here: https://support.illumina.com/array/array kits/infinium-methylationepic-beadchip-kit/downloads.html..

The methylation level of a particular CpG site can be represented by its M-value, which is the log2 ratio of the intensities of methylated probe versus unmethylated probe. Hence, positive M-values mean that more molecules are methylated than unmethylated, while negative M-values mean the opposite.

## RNA expression analysis

RNA-Seq (e.g. using Illumina's® TruSeq RNA Library Prep Kit and HiSeq system), RT-qPCR, SAGE, EST sequencing, or hybridisation-based methods such as microarrays may be used to sequence the whole transcriptome or to determine the expression level of individual RNAs or sets of RNAs.

As used herein, "an RNA" means a particular sequence of ribonucleic acid. "RNA" includes both coding mRNAs and non-coding RNAs. As used herein, "a transcript" means a single molecule of an RNA.

The expression level of a particular RNA can be measured in transcripts per million (TPM). A value of x TPM means that for every 1 million transcripts (RNA molecules) in the sample, x are transcripts of the gene of interest. Gene expression level statistics may be generated by summing up all transcripts of a given gene.

## Similarity networks

"Similarity networks", also referred to herein as "subject similarity networks" or "patient similarity networks", can be used to cluster or classify subjects based on their similarities in various features (Pai and Bader (2018) Patient Similarity Networks for Precision Medicine. J Mol Biol. 430:2924-2938).

In certain embodiments of the present invention, subject similarity networks may be generated based on methylation data (i.e. the methylation levels of CpG sites) or RNA expression data (i.e. the expression levels of RNAs). In certain embodiments of the present invention, subject similarity networks may be generated based on methylation data. In certain embodiments of the present invention, subject similarity networks may be generated based on RNA expression data.

Similarity network fusion (SNF) can be used to integrate multiple types of data into a single, fused similarity network. SNF comprises two main steps: (1) generating a subject (patient) similarity network for each data type (i.e. one for CpG methylation levels and one for RNA expression levels) and (2) integrating these networks into a single fused similarity network using a nonlinear combination method (provided in Wang et al. (2014) Similarity network fusion for aggregating data types on a genomic scale. Nat Methods. 11(3):333-7). Advantageously, fused similarity networks can represent the full spectrum of underlying data. To date, fused patient similarity networks have primarily been used to identify clinically homogenous patient subgroups in cancers.

Similarity network fusion is advantageous over other methods of data integration because it works well with relatively low numbers of samples, it is robust to noise, it scales well to very high numbers of features, and/or it is computationally efficient even with larger numbers of different data levels that are to be integrated.

Hence, in certain embodiments of the present invention, fused subject similarity networks may be generated based on both methylation data and RNA expression data. In other words, fused subject similarity networks may be generated based on the methylation levels of CpG sites and the expression levels of RNAs.

In certain embodiments of the present invention, similarity network fusion may be performed using parameter settings of $k$ = 10 - 30 (number of neighbors), $t$ = 10 - 20 (number of iterations), and/or $alpha$ = 0.3 - 0.8 (hyperparameter). In some embodiments, similarity network fusion may be performed using parameter settings of $k$ = 10, $t$ = 20, and/or $alpha$ = 0.5.

**CpG sites and RNAs**

In certain embodiments, the set of CpG sites whose methylation levels are determined and/or used to generate the subject similarity network may comprise at least about 2,500, at least about 5,000, at least about 10,000, at least about 20,000, at least about 40,000, or at least about 43,290 CpG sites. In certain embodiments, the set of CpG sites whose methylation levels are determined and/or used to generate the subject similarity network may comprise at least about 43,290 CpG sites. In certain embodiments, the set of CpG sites whose methylation levels are determined and/or used to generate the subject similarity network may comprise from about 2,500 to about 640,000 CpG sites, from about 5,000 to about 320,000 CpG sites, from about 10,000 to about 160,000 CpG sites, or from about 20,000 to about 80,000 CpG sites.

In certain embodiments, the set of CpG sites whose methylation levels are determined and/or used to generate the subject similarity network may comprise at least about 0.3%, at least about 0.6%, at least about 1.2%, at least about 2.5%, or at least about 5% of the CpG sites in the Infinium MethylationEPIC BeadChip array. In certain embodiments, the set of CpG sites whose methylation levels are determined and/or used to generate the subject similarity network may comprise at least about 5% of the CpG sites in the Infinium MethylationEPIC BeadChip array. In certain embodiments, the set of CpG sites whose methylation levels are determined and/or used to generate the subject similarity network may comprise from about 0.3% to about 80% of the CpG sites in the Infinium MethylationEPIC BeadChip array, from about 0.6% to about 40% of the CpG sites in the Infinium MethylationEPIC BeadChip array, from about 1.2% to about 20% of the CpG sites in the Infinium MethylationEPIC BeadChip array, or from about 2.5% to about 10% of the CpG sites in the Infinium MethylationEPIC BeadChip array.

In certain embodiments, the set of RNAs (transcripts) whose expression levels are determined and/or used to generate the subject similarity network may comprise at least about 100, at least about 200, at least about 400, at least about 800, or at least about 1600 RNAs. In certain embodiments, the set of RNAs whose expression levels are determined and/or used to generate the subject similarity network may comprise at least about 1,645 RNAs. In certain embodiments, the set of RNAs (transcripts) whose expression levels are determined and/or used to generate the subject similarity network may comprise from about 100 to about 25,000 RNAs, from about 200 to about 13,000 RNAs, from about 400 to about 6,500 RNAs, or from about 800 to about 3,200 RNAs.

In certain embodiments, the set of RNAs whose expression levels are determined and/or used to generate the subject similarity network may comprise at least about 0.3%, at least about 0.6%, at least about 1.2%, at least about 2.5%, or at least about 5% of the RNAs in the transcriptome of the skin cells. In certain embodiments, the set of RNAs whose expression levels are determined and/or used to generate the subject similarity network may comprise at least about 5% of the RNAs in the transcriptome of the skin cells. In certain embodiments, the set of RNAs (transcripts) whose expression levels are determined and/or used to generate the subject similarity network may comprise from about 0.3% to about 80% of the RNAs in the transcriptome of the skin cells, from about 0.6% to about 40% of the RNAs in the transcriptome of the skin cells, from about 1.2% to about 20% of the RNAs in the transcriptome of the skin cells, or from about 2.5% to about 10% of the RNAs in the transcriptome of the skin cells.

In certain embodiments of the present invention, the methylation levels of at least about 2,500, at least about 5,000, at least about 10,000, at least about 20,000, or at least about 40,000 CpG sites and the expression levels of at least about 100, at least about 200, at least about 400, at least about 800, or at least about 1600 RNAs may be determined and/or used to generate a subject similarity network.

For example, the methylation levels of at least about 40,000 CpG sites and the expression levels at least about 1,600 RNAs may be determined and/or used to generate a subject similarity network. In another example, the methylation levels of at least about 43,290 CpG sites and the expression levels at least about 1,645 RNAs may be determined and/or used to generate a subject similarity network.

In certain embodiments of the present invention, the methylation levels of at least about 0.3%, at least about 0.6%, at least about 1.2%, at least about 2.5%, or at least about 5% of the CpG sites in the Infinium MethylationEPIC BeadChip array and the expression levels of at least about 0.3%, at least about 0.6%, at least about 1.2%, at least about 2.5%, or at least about 5% of the RNAs in the transcriptome of the skin cells may be determined and/or used to generate a subject similarity network.

For example, the methylation levels of at least about 2.5% of the CpG sites in the Infinium MethylationEPIC BeadChip array and the expression levels at least about 2.5% of the RNAs in the transcriptome of the skin cells may be determined and/or used to generate a subject similarity network. In another example, the methylation levels of at least about 5% of the CpG sites in the Infinium MethylationEPIC BeadChip array and the expression levels at least about 5% of the RNAs in the transcriptome of the skin cells may be determined and/or used to generate a subject similarity network.

In certain embodiments of the present invention, the CpG sites whose methylation levels are determined and/or used to generate the subject similarity network may be CpG sites whose methylation levels exhibit a correlation with MED. For example, the CpG sites whose methylation levels are determined and/or used to generate the subject similarity network may fall within the 1%, 2%, 5%, 10%, or 20% of CpG sites in the Infinium MethylationEPIC BeadChip array whose methylation levels correlate most strongly with MED. In some embodiments, the CpG sites whose methylation levels are determined and/or used to generate the subject similarity network may fall within the 5% of CpG sites in the Infinium MethylationEPIC BeadChip array whose methylation levels correlate most strongly with MED.

Similarly, in certain embodiments of the present invention, the RNAs whose expression levels are determined and/or used to generate the subject similarity network may be RNAs whose expression levels exhibit a correlation with MED. For example, the RNAs whose expression levels are determined and/or used to generate the subject similarity network may fall within the 1%, 2%, 5%, 10%, or 20% of RNAs whose expression levels correlate most strongly with MED. In some embodiments, the RNAs whose expression levels are determined and/or used to generate the subject similarity network may fall within the 5% of RNAs in the transcriptome whose expression levels correlate most strongly with MED.

Hence, in certain embodiments, methods of the present invention may comprise a step of determining, obtaining, or receiving the subjects' MEDs. In certain embodiments, methods of the present invention may comprise a step of identifying the 1%, 2%, 5%, 10%, or 20% of CpG sites and/or RNAs whose methylation levels and/or expression levels, respectively, correlate most strongly with MED. In some embodiments, methods of the present invention may comprise a step of identifying the 5% of CpG sites and/or RNAs whose methylation levels and/or expression levels, respectively, correlate most strongly with MED. As used herein, "correlation" refers to absolute Pearson or Spearman correlation coefficient.

**Network clustering**

In certain embodiments of the present invention, once a similarity network (optionally a fused similarity network) has been generated, cluster analysis may be used to identify highly interconnected clusters within the network. Cluster analysis may be performed using spectral clustering (previously described in Wang et al. (2014) Similarity network fusion for aggregating data types on a genomic scale. Nat Methods. 11(3):333-7), Hierarchical clustering, k-means, k-medoids, expectation-maximization, affinity propagation, density-based clustering, self-organizing maps, or community detection algorithms (e.g. louvain clustering). In some embodiments, spectral clustering may be used.

**Molecular Phototypes**

The present invention is based in part on the surprising finding that when human subjects are clustered based on the similarity of the methylation status of CpG sites and/or the expression level of RNAs in their skin cells in response to UV irradiation, the subjects cluster into discrete subgroups. In the present application, "subgroups" may also be referred to as subtypes, phototypes, or "Molecular Phototypes".

The methods described herein can be used for classifying humans into Molecular Phototypes based on the biological responses (methylation and/or RNA expression) of their skin to UV irradiation. As used herein, "classifying" means categorising, subtyping, dividing, or stratifying subjects into subgroups.

In certain embodiments, the methods of the present invention may involve defining Molecular Phototypes based on the clusters in the subject similarity network. In other words, in certain embodiments, the methods of the present invention may involve defining each cluster on a similarity network as a distinct Molecular Phototype. For example, if the similarity network shows 3 clusters, subjects belonging to a first cluster may be classified as "Molecular Phototype 1", subjects belonging to a second cluster may be classified as "Molecular Phototype 2", and subjects belonging to a third cluster may be classified as "Molecular Phototype 3".

## Characterising Molecular Phototypes

The present invention is based in part on the finding that subjects belonging to the discrete Molecular Phototypes have divergent biological responses to UV irradiation of their skin cells.

Hence, the present invention relates to methods for identifying biological pathways that are associated with the response to UV irradiation of skin in human subjects belonging to a Molecular Phototype determined by a method described herein. A biological pathway is "associated with the response to UV irradiation" if one or more genes annotated in that pathway are responsive to UV irradiation. Hence, the present invention also relates to methods for identifying one or more genes that are responsive to UV irradiation of skin in human subjects belonging to a Molecular Phototype determined by a method described herein. A gene is "responsive to UV irradiation" if its expression is up- or down-regulated in response to said UV irradiation.

Such methods may comprise providing skin cells irradiated with UV light and control skin cells obtained from a plurality of human subjects, as described above, wherein the subjects belong to a single, discrete Molecular Phototype determined by a method described herein.

Such methods may further comprise determining the expression levels of at least one gene in the said irradiated and control skin cells, wherein said at least one gene is annotated to a biological pathway. As used herein, a gene "annotated to" a biological pathway may also be referred to as a gene "involved in" a biological pathway or a gene "belonging to" a biological pathway.

In certain embodiments, the method may comprise determining the expression level of a single gene in the said irradiated and control skin cells. In other words, a gene-based approach may be used.

Alternatively, the method may comprise determining the expression level of a set of genes in the said irradiated and control skin cells. In certain embodiments, the set of genes may be known to be associated with a biological pathway or process. In other words, a pathway- or process- based approach may be used. Known databases provide sets of genes associated with particular biological pathways or processes. For example, GO term gene sets, WikiPathways, KEGG, Biocarta and Reactome pathways, the Hallmark Gene Set Collection and/or gene sets such as those included in the Molecular Signature Database v7.0 (Liberzon et al. (2011) Molecular signatures database (MSigDB) 3.0. Bioinformatics 27:1739-1740; http://software.broadinstitute.org/gsea/msigdb/genesets.jsp) may be used.

In certain embodiments, the methods may further comprise using a machine learning model to determine whether the gene(s) are able to discriminate between irradiated and control skin cells. In other words, the methods may further comprise using a machine learning model to determine whether the gene(s) are able to predict irradiation status of samples.

The methods of the invention may further comprise determining the strength of the association between the biological pathway and the response to UV irradiation of skin in the human subjects belonging to the Molecular Phototype, wherein the strength of the association is based on the degree to which the machine-learning model can discriminate between irradiated and control skin cells. In certain embodiments, the biological pathway(s) may be identified as being associated with the skin's response to UV irradiation in human subjects belonging to the Molecular Phototype being tested if the at least one gene is able to discriminate between irradiated and control skin cells or is able to predict irradiation status of samples to a certain accuracy level, for example at least about 70, at least about 80, at least about 90, or at least about 95% accuracy.

In certain embodiments of the present invention, the similarity network shows 3 clusters. In certain embodiments, a first cluster may be characterised by differential regulation of biological pathways relating to inflammasome activation, interleukin and general cytokine response, protein modification, unfolded protein response, lipid biosynthetic and catabolic processes, regulation of cell adhesion, steroid hormone receptor activity, necrotic cell death, macrophage activation, activation of positive chemotaxis, phagocytosis, and/or response to pain. In certain embodiments, a second cluster may be characterised by differential regulation of biological pathways relating to type I interferon response, spliceosome, termination of polymerase II transcription regulation of stem cell population maintenance, histone demethylase activity, response to vitamin D, regulation of DNA methylation, snRNA processing, negative regulation of cell proliferation, regulation of cellular senescence, protein import into mitochondrial matrix, catabolism of misfolded protein, nonsense mediated mRNA decay, mitochondrial morphogenesis and transport, and/or cell differentiation. In certain embodiments, a third cluster may be characterised by differential regulation of biological pathways relating to cell cycle checkpoints, DNA synthesis, chromosome condensation, oxidoreduction coenzyme metabolic process response, tRNA modification, reverse cholesterol transport, nucleotide salvage, water homeostasis, DNA packaging, histone phosphorylation, and/or maintenance of epithelial cell polarity.

## Machine learning models

In the context of the present invention, "machine learning models" may also be referred to as "machine learning classifiers".

In certain embodiments of the present invention, the machine learning model may be trained to discriminate between irradiated and control samples using expression data of a gene or set of genes (e.g. a pathway) in subjects belonging to a single, discrete Molecular Phototype. In certain embodiments of the present invention, the machine learning model may be trained to predict irradiation status of a sample using expression data of a gene or set of genes (e.g. a pathway) in subjects belonging to a single, discrete Molecular Phototype.

In certain embodiments, the machine learning model may have been trained on data comprising irradiation status (i.e. irradiated or control) and expression levels of the selected gene(s). In certain embodiments, the models may have been trained on a data set comprising data from at least 10, 20, 30, or 32 subjects.

In certain embodiments of the present invention, the machine learning models may be support vector machines (SVMs). In further embodiments, the machine learning models may be logistic regression models, decision trees, random forests, gradient boosting, or artificial neural networks.

In certain embodiments, the SVMs may use radial basis function kernels. The hyperparameters may be set to

$$gamma = \frac{1}{size\ of\ gene\ set}$$

and $C$ may be from 0 to about 100, from 0 to about 10, or about 1. Optionally,

$$gamma = \frac{1}{size\ of\ gene\ set}$$

and $C = 1$.

In certain embodiments of the present invention, the machine learning model may be scored for how well it enables discrimination between irradiated and control samples in a repeated cross-validation scheme. In further embodiments, the machine learning model may be scored for how well it enables discrimination between irradiated and control samples using a simple training-test split, cross-validation without repetitions, leave-one-out-cross-validation, or out-of-bag bootstrapping.

In other words, a machine learning model may be scored for how accurately it predicts irradiation status in a repeated cross-validation scheme. In examples, a 5 x 5 fold repeated cross-validation may be used.

## Computer programs

The present invention further provides a computer program for classifying humans into Molecular Phototypes based on the biological response of their skin to UV irradiation, the computer program comprising instructions which, when the program is executed by a computer, cause the computer to carry out the following steps:

a) inputting the methylation levels and/or expression levels of at least about 100 features in skin cells irradiated with UV light obtained from a plurality of human subjects, wherein the features are selected from CpG sites and RNAs;

b) using the methylation data and/or expression data obtained in step b) to generate a subject similarity network;

c) performing cluster analysis on the subject similarity network; and

d) outputting definitions of Molecular Phototypes based on the clusters in the subject similarity network.

The present invention further provides a computer program for identifying biological pathways that are associated with the response to UV irradiation of skin in human subjects belonging to a Molecular Phototype determined by a method described herein, the computer program comprising instructions which, when the program is executed by a computer, cause the computer to carry out the following steps:

a) inputting the expression level of at least one gene annotated to a biological pathway obtained from skin cells irradiated with UV light and control skin cells obtained from a plurality of human subjects belonging to a Molecular Phototype determined by a method described herein;

b) using a machine-learning model trained on the data obtained in step a) to identify whether said at least one gene can discriminate between irradiated and control skin cells;

c) determining the strength of the association between the biological pathway and the response to UV irradiation of skin in the human subjects belonging to the Molecular Phototype, wherein the strength of the association is based on the degree to which the machine-learning model can discriminate between irradiated and control skin cells; and

d) outputting a decision on whether the biological pathway is associated with the response to UV irradiation of skin in human subjects belonging to the Molecular Phototype.

The present invention further provides a computer-readable medium comprising one or more of the computer programs described above.

For the avoidance of doubt, the present application is directed to subject-matter described in the following numbered paragraphs:

1. A method for classifying humans into Molecular Phototypes based on the biological response of their skin to UV irradiation, the method comprising:

a) providing skin cells irradiated with UV light obtained from a plurality of human subjects;

b) determining the methylation levels and/or expression levels of at least about 100 features in said skin cells, wherein the features are selected from CpG sites and RNAs;

c) using the methylation data and/or expression data obtained in step b) to generate a subject similarity network;

d) performing cluster analysis on the subject similarity network; and

e) defining Molecular Phototypes based on the clusters in the subject similarity network.

2. The method of paragraph 1, wherein step b) comprises determining the methylation levels of:

i) at least about 2,500, at least about 5,000, at least about 10,000, at least about 20,000, or at least about 40,000 CpG sites; or

ii) at least about 0.3%, at least about 0.6%, at least about 1.2%, at least about 2.5%, or at least about 5% of the CpG sites in the Infinium MethylationEPIC BeadChip array.

3. The method of paragraph 1 or 2, wherein step b) comprises determining the expression levels of:

i) at least about 100, at least about 200, at least about 400, at least about 800, or at least about 1,600 RNAs; or

ii) at least about 0.3%, at least about 0.6%, at least about 1.2%, at least about 2.5%, or at least about 5% of the RNAs in the transcriptome of the skin cells.

4. The method of any preceding paragraph, wherein step b) comprises determining the methylation level of at least one CpG site and determining the expression level of at least one RNA.

5. The method of paragraph 4, wherein step c) comprises:

i) generating a subject similarity network for the CpG sites methylation data obtained in step b);

ii) generating a subject similarity network for the RNA expression data obtained in step b); and

iii) integrating the two subject similarity networks into a single, fused similarity network.

6. The method of paragraph 5, wherein the similarity network fusion is performed using parameter settings wherein $k$ is from about 10 to about 30, $t$ is from about 10 to about 20, and/or $alpha$ is from about 0.3 to about 0.8.

7. The method of any preceding paragraph, wherein step b) comprises determining the methylation levels of:

i) at least about 2,500, at least about 5,000, at least about 10,000, at least about 20,000, or at least about 40,000 CpG sites; or

ii) at least about 0.3%, at least about 0.6%, at least about 1.2%, at least about 2.5%, or at least about 5% of the CpG sites in the Infinium MethylationEPIC BeadChip array,

and determining the expression levels of:

i) at least about 100, at least about 200, at least about 400, at least about 800, or at least about 1,600 RNAs; or

ii) at least about 0.3%, at least about 0.6%, at least about 1.2%, at least about 2.5, or at least about 5% of the RNAs in the transcriptome of the skin cells.

8. The method of any preceding paragraph, wherein the CpG sites' methylation levels and/or the RNAs' expression levels correlate with MED.

9. The method of any preceding paragraph, wherein the CpG sites fall in the 1%, 5%, 10%, or 20% of CpG sites in the Infinium MethylationEPIC BeadChip array whose methylation levels correlate most strongly with MED and/or the RNAs fall in the 1%, 5%, 10%, or 20% of RNAs in the transcriptome whose expression levels correlate most strongly with MED.

10. The method of any preceding paragraph, wherein the cluster analysis in step d) is performed using spectral clustering, Hierarchical clustering, k-means, k-medoids, expectation-maximization, affinity propagation, density-based clustering, self-organizing maps, or community detection algorithms, optionally louvain clustering.

11. The method of any preceding paragraph, wherein the skin cells comprised as a majority, consisted essentially of, or consisted of epidermis cells and/or dermis cells.

12. The method of any preceding paragraph, wherein the skin cells have been irradiated with an amount of UV light that achieved an MED of from about 0.5 to about 1.3, from about 0.7 to about 1.1, from about 0.8 to about 1.0, or about 0.9.

13. The method of any preceding paragraph, wherein the skin cells have been irradiated at least 2 times, at least 3 times, from 2 to 4 times, or 3 times.

14. The method of paragraph 13, wherein the irradiations have been performed 12 hours to about 48 hours apart, about 12 hours to about 36 hours apart, about 20 to about 28 hours apart, or about 24 hours apart.

15. The method of any preceding paragraph, wherein the subjects are Caucasian and/or belong to Fitzpatrick phototype I-IV.

16. The method of any preceding paragraph, wherein the subject similarity network identifies three distinct clusters, which are designated as three Molecular Phototypes.

17. The method of paragraph 16, wherein:

i) a first cluster is characterised by differential regulation of biological pathways relating to inflammasome activation, interleukin and general cytokine response, protein modification, unfolded protein response, lipid biosynthetic and catabolic processes, regulation of cell adhesion, steroid hormone receptor activity, necrotic cell death, macrophage activation, activation of positive chemotaxis, phagocytosis, and/or response to pain;
ii) a second cluster is characterised by differential regulation of biological pathways relating to type I interferon response, spliceosome, termination of polymerase II transcription regulation of stem cell population maintenance, histone demethylase activity, response to vitamin D, regulation of DNA methylation, snRNA processing, negative regulation of cell proliferation, regulation of cellular senescence, protein import into mitochondrial matrix, catabolism of misfolded protein, nonsense mediated mRNA decay, mitochondrial morphogenesis and transport, and/or cell differentiation; and/or
iii) a third cluster is characterised by differential regulation of biological pathways relating to cell cycle checkpoints, DNA synthesis, chromosome condensation, oxidoreduction coenzyme metabolic process response, tRNA modification, reverse cholesterol transport, nucleotide salvage, water homeostasis, DNA packaging, histone phosphorylation, and/or maintenance of epithelial cell polarity.

18. A computer program for classifying humans into Molecular Phototypes based on the biological response of their skin to UV irradiation, the computer program comprising instructions which, when the program is executed by a computer, cause the computer to carry out the following steps:

a) inputting the methylation levels and/or expression levels of at least about 100 features in skin cells irradiated with UV light obtained from a plurality of human subjects, wherein the features are selected from CpG sites and RNAs;

b) using the methylation data and/or expression data obtained in step b) to generate a subject similarity network;
c) performing cluster analysis on the subject similarity network; and
d) outputting definitions of Molecular Phototypes based on the clusters in the subject similarity network.

19. The computer program of paragraph 18, wherein step a) comprises inputting the methylation levels of:

i) at least about 2,500, at least about 5,000, at least about 10,000, at least about 20,000, or at least about 40,000 CpG sites; or
ii) at least about 0.3%, at least about 0.6%, at least about 1.2%, at least about 2.5%, or at least about 5% of the CpG sites in the Infinium MethylationEPIC BeadChip array.

20. The computer program of paragraph 18 or 19, wherein step a) comprises inputting the expression levels of:

i) at least about 100, at least about 200, at least about 400, at least about 800, or at least about 1,600 RNAs; or
ii) at least about 0.3%, at least about 0.6%, at least about 1.2%, at least about 2.5%, or at least about 5% of the RNAs in the transcriptome of the skin cells.

21. The computer program of any of paragraphs 18-20, wherein step a) comprises inputting the methylation level of at least one CpG site and inputting the expression level of at least one RNA.

22. The computer program of paragraph 21, wherein step b) comprises:

i) generating a subject similarity network for the CpG sites methylation data inputted in step a);
ii) generating a subject similarity network for the RNA expression data inputted in step a); and
iii) integrating the two subject similarity networks into a single, fused similarity network.

23. The computer program of paragraph 22, wherein the similarity network fusion is performed using parameter settings wherein *k* is from about 10 to about 30, *t* is from about 10 to about 20, and/or *alpha* is from about 0.3 to about 0.8.

24. The computer program of any of paragraphs 18-23, wherein step a) comprises inputting the methylation levels of:

i) at least about 2,500, at least about 5,000, at least about 10,000, at least about 20,000, or at least about 40,000 CpG sites; or

ii) at least about 0.3%, at least about 0.6%, at least about 1.2%, at least about 2.5%, or at least about 5% of the CpG sites in the Infinium MethylationEPIC BeadChip array,

and inputting the expression levels of:

iii) at least about 100, at least about 200, at least about 400, at least about 800, or at least about 1,600 RNAs; or

iv) at least about 0.3%, at least about 0.6%, at least about 1.2%, at least about 2.5, or at least about 5% of the RNAs in the transcriptome of the skin cells.

25. The computer program of any of paragraphs 18-24, wherein the CpG sites' methylation levels and/or the RNAs' expression levels correlate with MED.

26. The computer program of any of paragraphs 18-25, wherein the CpG sites fall in the 1%, 5%, 10%, or 20% of CpG sites in the Infinium MethylationEPIC BeadChip array whose methylation levels correlate most strongly with MED and/or the RNAs fall in the 1%, 5%, 10%, or 20% of RNAs in the transcriptome whose expression levels correlate most strongly with MED.

27. The computer program of any of paragraphs 18-26, wherein the cluster analysis in step c) is performed using spectral clustering, Hierarchical clustering, k-means, k-medoids, expectation-maximization, affinity propagation, density-based clustering, self-organizing maps, or community detection algorithms, optionally louvain clustering.

28. The computer program of any of paragraphs 18-27, wherein the skin cells comprised as a majority, consisted essentially of, or consisted of epidermis cells and/or dermis cells.

29. The computer program of any of paragraphs 18-28, wherein the skin cells had been irradiated with an amount of UV light that achieved an MED of from about 0.5 to about 1.3, from about 0.7 to about 1.1, from about 0.8 to about 1.0, or about 0.9.

30. The computer program of any of paragraphs 18-29, wherein the skin cells had been irradiated at least 2 times, at least 3 times, from 2 to 4 times, or 3 times.

31. The computer program of paragraph 30 when the irradiations had been performed 12 hours to about 48 hours apart, about 12 hours to about 36 hours apart, about 20 to about 28 hours apart, or about 24 hours apart.

32. The computer program of any of paragraphs 18-31, wherein the subjects were Caucasian and/or belonged to Fitzpatrick phototype I-IV.

33. The computer program of any of paragraphs 18-32, wherein the subject similarity network identifies three distinct clusters, which are outputted as three Molecular Phototypes based on the three clusters.

34. The computer program of paragraph 33, wherein the computer program outputs one or more of the following definitions of Molecular Phototypes:

i) a first Molecular Phototype characterised by differential regulation of biological pathways relating to inflammasome activation, interleukin and general cytokine response, protein modification, unfolded protein response, lipid biosynthetic and catabolic processes, regulation of cell adhesion, steroid hormone receptor activity, necrotic cell death, macrophage activation, activation of positive chemotaxis, phagocytosis, and/or response to pain;

ii) a second Molecular Phototype characterised by differential regulation of biological pathways relating to type I interferon response, spliceosome, termination of polymerase II transcription regulation of stem cell population maintenance, histone demethylase activity, response to vitamin D, regulation of DNA methylation, snRNA processing, negative regulation of cell proliferation, regulation of cellular senescence, protein import into mitochondrial matrix, catabolism of misfolded protein, nonsense mediated mRNA decay, mitochondrial morphogenesis and transport, and/or cell differentiation; and/or

iii) a third Molecular Phototype characterised by differential regulation of biological pathways relating to cell cycle checkpoints, DNA synthesis, chromosome condensation, oxidoreduction coenzyme metabolic process response, tRNA modification, reverse cholesterol transport, nucleotide salvage, water homeostasis, DNA packaging, histone phosphorylation, and/or maintenance of epithelial cell polarity.

35. A computer-readable medium comprising the computer program of any of paragraphs 18-34.

36. A method for identifying biological pathways that are associated with the response to UV irradiation of skin in human subjects belonging to a Molecular Phototype as determined by any of paragraphs 1-35, the method comprising:

a) providing skin cells irradiated with UV light and control skin cells obtained from a plurality of human subjects belonging to a Molecular Phototype determined by the method of any of paragraphs 1-35;

b) determining the expression levels of at least one gene in said irradiated and control skin cells, wherein said at least one gene is annotated to a biological pathway;

c) using a machine-learning model trained on the data obtained in step b) to identify whether said at least one gene can discriminate between irradiated and control skin cells; and

d) determining the strength of the association between the biological pathway and the response to UV irradiation of skin in the human subjects belonging to the Molecular Phototype, wherein the strength of the association is based on the degree to which the machine-learning model can discriminate between irradiated and control skin cells.

37. The method of paragraph 36, wherein the at least one gene is a set of genes annotated to a biological pathway.

38. The method of paragraph 37, wherein the set of genes annotated to a biological pathway is selected from the sets of genes in:

    i) the GO term collection;
    ii) the WikiPathways collection;
    iii) the KEGG pathway collection;
    iv) the BioCarta pathway collection;
    v) the Reactome pathway collection; and/or
    vi) the Hallmark gene set collection.

39. The method of any of paragraphs 36-38, wherein the biological pathway is identified as being associated with the skin's response to UV irradiation in human subjects belonging to the Molecular Phototype if the at least one gene is able to discriminate between irradiated and control skin cells with at least about 70, at least about 80, at least about 90, or at least about 95% accuracy.

40. The method of any of paragraphs 36-39, wherein the machine learning model has been trained on data comprising irradiation statuses and expression levels of the at least one gene.

41. The method of any of paragraphs 36-40, wherein the machine learning model is a support vector machine (SVM), a logistic regression model, a decision tree, a random forest, gradient boosting, or an artificial neural network.

42. The method of any of paragraphs 36-41, wherein the skin cells comprise as a majority, consist essentially of, or consist of epidermis cells and/or dermis cells.

43. The method of any of paragraphs 36-42, wherein the UV irradiated skin cells have been irradiated with an amount of UV light that achieved an MED of from about 0.5 to about 1.3, from about 0.7 to about 1.1, from about 0.8 to about 1.0, or about 0.9.

44. The method of any of paragraphs 36-43, wherein the UV irradiated skin cells have been irradiated at least 2 times, at least 3 times, from 2 to 4 times, or 3 times.

45. The method of paragraph 44, when the irradiations have been performed 12 hours to about 48 hours apart, about 12 hours to about 36 hours apart, about 20 to about 28 hours apart, or about 24 hours apart.

46. A computer program for identifying biological pathways that are associated with the response to UV irradiation of skin in human subjects belonging to a Molecular Phototype as determined by any of paragraphs 1-35, the computer program comprising instructions which, when the program is executed by a computer, cause the computer to carry out the following steps:

    a) inputting the expression levels of at least one gene obtained from skin cells irradiated with UV light and control skin cells obtained from a plurality of human subjects belonging to a Molecular Phototype determined by the method of any of paragraphs 1-35, wherein the at least one gene is annotated to a biological pathway;

    b) using a machine-learning model trained on the data obtained in step a) to identify whether said at least one gene can discriminate between irradiated and control skin cells;

    c) determining the strength of the association between the biological pathway and the response to UV irradiation of skin in the human subjects belonging to the Molecular Phototype, wherein the strength of the association is based on the degree to which the machine-learning model can discriminate between irradiated and control skin cells; and

    d) outputting a decision on whether the biological pathway is associated with the response to UV irradiation of skin in human subjects belonging to the Molecular Phototype.

47. The computer program of paragraph 46, wherein the at least one gene is a set of genes annotated to a biological pathway.

48. The computer program of paragraph 47, wherein the set of genes annotated to a biological pathway is selected from:

   i) the GO term collection;
   ii) the WikiPathways collection;
   iii) the KEGG pathway collection;
   iv) the BioCarta pathway collection;
   v) the Reactome pathway collection; and/or
   vi) the Hallmark gene set collection.

49. The computer program of any of paragraphs 46-48, wherein the biological pathway is identified as being associated with the skin's response to UV irradiation in human subjects belonging to the Molecular Phototype if the at least one gene is able to discriminate between irradiated and control skin cells with at least about 70, at least about 80, at least about 90, or at least about 95% accuracy.

50. The computer program of any of paragraphs 46-49, wherein the machine learning model has been trained on data comprising irradiation statuses and expression levels of the at least one gene.

51. The computer program of any of paragraphs 46-50, wherein the machine learning model is a support vector machine (SVM), a logistic regression model, a decision tree, a random forest, gradient boosting, or an artificial neural network.

52. The computer program of any of paragraphs 46-51, wherein the skin cells comprised as a majority, consisted essentially of, or consisted of epidermis cells and/or dermis cells.

53. The computer program of any of paragraphs 46-52, wherein the UV irradiated skin cells had been irradiated with an amount of UV light that achieved an MED of from about 0.5 to about 1.3, from about 0.7 to about 1.1, from about 0.8 to about 1.0, or about 0.9.

54. The computer program of any of paragraphs 46-53, wherein the UV irradiated skin cells had been irradiated at least 2 times, at least 3 times, from 2 to 4 times, or 3 times.

55. The computer program of paragraphs 54, when the irradiations had been performed 12 hours to about 48 hours apart, about 12 hours to about 36 hours apart, about 20 to about 28 hours apart, or about 24 hours apart.

It should be noted that the present invention may comprise any combination of features and/to limitations referred to herein, except for combinations of such features which are mutually exclusive. The foregoing description is directed to particular embodiments of the present invention for the purpose of illustrating it. It will be apparent, however, to one skilled in the art, that many modifications and variations to the embodiments described herein are possible. All such modifications and variations are intended to be within the scope of the present invention, as defined in the appended claims.

## EXAMPLES

### Methods

### UV irradiation

MED was determined as previously described (Heckman et al (2013) Minimal Erythema Dose (MED) Testing, J Vis Exp. (75): 50175) and outlined below. This method has been standardised as International Standard ISO 2444:2010.

The study sites were located on the subjects' lower backs since this area is rarely exposed to sunlight. The sites were split into control and test areas.

The first irradiation of the test sites was performed using a SOL 500 full spectrum solar simulator (Hönle UV Technology). Intensities were chosen individually to reach 0.9 MED for all subjects (i.e. 90% of the MED in a given test subject).

Irradiation to 0.9 MED was repeated twice more in the same manner, each time 24 hours apart, so that all test sites had been irradiated three times.

### Skin sampling

24 hours after the final irradiation session of each subject, four suction blisters of 7 mm diameter were taken from both test sites (irradiated) and control sites (not irradiated), as previously described (Südel et al. (2003) Tight control of matrix metalloproteinase-1 activity in human skin. Photochem Photobiol. 78: 355-60).

A single sample of the epidermis from each test site was sufficient to perform transcriptome/RNA sequencing and methylation profiling, as described herein.

### Nucleic acid extraction

Tissue samples were suspended in the respective lysis buffers for RNA or DNA extraction and homogenized using an MM 301 bead mill (Retsch®). DNA was then extracted using the QIAamp® DNA Investigator Kit (Qiagen®) according to manufacturer's instructions. RNA was extracted using the RNeasy® Fibrous Tissue Mini Kit (Qiagen®) according to manufacturer's instructions. RNA and DNA samples were used for transcriptome sequencing and methylation profiling, respectively, as described below.

**Methylation profiling**

Methylation profiling was performed using Illumina's® Infinium Methylation EPIC arrays (Love et al. (2014) Moderated estimation of fold change and dispersion for RNA-seq data with DESeq2, Genome Biol. 15:550).

Methylation data was processed using the minfi package (Aryee (2014) Minfi: a flexible and comprehensive Bioconductor package for the analysis of Infinium DNA methylation microarrays, Bioinformatics 30:1363-1369) in R. Normalization was carried out using the funnorm normalization method.

This method determined the methylation level of over 850000 human CpG sites throughout the genome with single nucleotide resolution.

**Transcriptome sequencing**

Transcriptome libraries were prepared using TruSeq Library Prep Kit (Illumina®) and sequencing performed at 1x50 bp on Illumina's® HiSeq system to a final sequencing depth of 100 million reads per sample.

Sequencing data was processed using a custom pipeline including Fastqc v0.11.767 for quality control, Trimmomatic v0.3668 for trimming and Salmon v0.8.169 for read mapping and quantification.

This method determined the expression level of over 35000 different RNAs.

**GO term enrichment analysis**

Enrichment analyses were performed using the z-score method (Lee et al. (2008) Inferring Pathway Activity toward Precise Disease Classification. PLoS Comput Biol. 4:e1000217) as implemented in the GSVA R package (Hänzelmann et al. (2013) GSVA: gene set variation analysis for microarray and RNA-seq data. BMC Bioinformatics. 14:7) GO term gene sets (Liberzon et al. (2015) The Molecular Signatures Database Hallmark Gene Set Collection. Cell Syst. 1:417-425) were downloaded from the Molecular Signatures Database v6.2 (Liberzon et al. (2011) Molecular signatures database (MSigDB) 3.0. Bioinformatics 27:1739-1740).

**Similarity network fusion and clustering**

For each type of feature (CpGs or RNAs), a filtering step was performed, in which the number of features was reduced to approximately 5% of the original number. In more detail, the inventors determined the correlation (Pearson's) of each feature with MED and ranked the features according to the strength of the correlation. The top 5% of features (i.e. the 5% of features that correlated most strongly with MED) were selected. This step may reduce technical bias.

CpG methylation data (M values) and RNA expression data (log2 transformed transcripts per million) from irradiated samples were integrated via similarity network fusion as previously described (Wang et al. (2014) Similarity network fusion for aggregating data types on a genomic scale. Nat Methods. 11:333-337) using parameter settings of $k$ = 10 (number of neighbors), $t$ = 20 (number of iterations) and *alpha* = 0.5 (hyperparameter).

Clustering on the fused network was then performed via spectral clustering as previously described (Wang et al. (2014) Similarity network fusion for aggregating data types on a genomic scale. Nat Methods. 11:333-337).

**Gene/pathway predictivity analysis**

Pathway predictivity analysis was performed using GO term gene sets (Liberzon et al. (2015) The Molecular Signatures Database Hallmark Gene Set Collection. Cell Syst. 1:417-425) downloaded from the Molecular Signatures Database v6.2 (Liberzon et al. (2011) Molecular signatures database (MSigDB) 3.0. Bioinformatics 27:1739-1740).

The pathway models were based on the support vector machine (SVM) implementation from the e1071 R package (Dimitriadou et al. (2011) e1071: Misc Functions of the Department of Statistics (e1071), TU Wien. R package version 1), interfaced via the mlr (Bischl et al. (2016) mlr: Machine Learning in R. J Mach Learn Res. 17:1-5) machine learning framework.

The models were trained by restricting the expression data to that of genes annotated within a given pathway and trained to predict sample irradiation status (control or irradiated to 0.9 MED) stratified by Molecular Phototype. The SVMs used the radial basis function kernel, with hyperparameters set to

$$gamma = \frac{1}{size\ of\ gene\ set}$$ and $C$ = 1.

Accuracy of prediction was derived from 5 x 5-fold repeated cross-validation for each pathway model, giving insight on how well genes within the gene set allow a discrimination between UV irradiated and control samples while controlling for overfitting, and used as a measure of predictivity of the respective pathway to irradiation status.

**EXAMPLE 1** - **classifying humans into Molecular Photototypes**

This study recruited 32 healthy female subjects belonging to Fitzpatrick phototypes I to IV (12 subjects belonging to phototype I and II, 10 to phototype III; and 10 to phototype IV). The subjects were aged between 30 and 65 years, with homogeneous age distributions in each Fitzpatrick phototype group.

Possible recruits were excluded is they had tattoos or scars in the area of skin to be exposed to UV radiation, if they had pigmentation disorders, if they were pregnant, or if they had taken anti-histamine or anti-inflammatory medication within two weeks prior to study start.

UV-irradiated (test) and non-irradiated (control) skin samples were obtained from each subject as described above. Nucleic acid was extracted and methylation profiling and transcriptome sequencing were performed, as described above. Similarity network fusion was used to integrate the methylation data and RNA expression data into a single, fused subject similarity network and spectral clustering was performed, as described above.

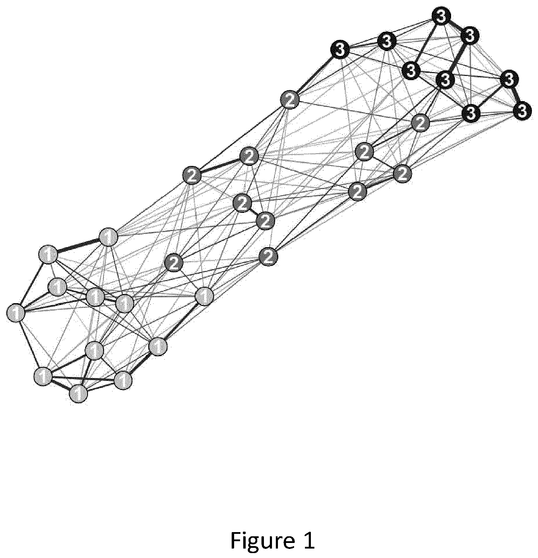

As shown in Figure 1, three discrete clusters were identified in the resulting fused similarity network. These clusters were designated Molecular Phototypes 1, 2, and 3.

**EXAMPLE 2 - characterising Molecular Phototypes by associated biological responses**

Each Molecular Phototype was characterised using GO term pathway-based predictivity analysis as described above.

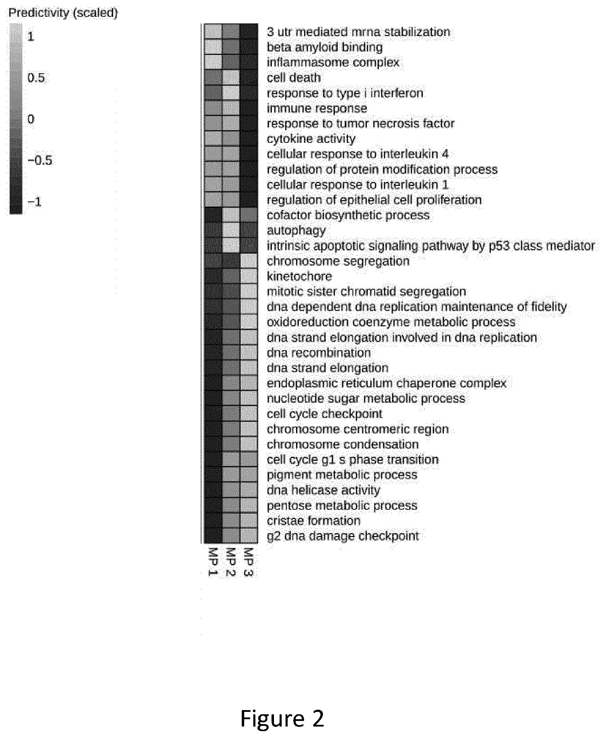

Analysis revealed divergent patterns between the three molecular subtypes, as shown in Figure 2.

MP1 and MP2 exhibited stronger signals in pathways associated with inflammatory and immune signalling in comparison to MP3.

MP1 exhibited strong signals in pathways related to inflammasome activation and interleukin and general cytokine response, protein modification, unfolded protein response, lipid biosynthetic and catabolic processes, regulation of cell adhesion, steroid hormone receptor activity, necrotic cell death, macrophage activation, activation of positive chemotaxis, phagocytosis, and response to pain.

MP2 exhibited decreased inflammasome scores but a stronger type I interferon response than either MP1 or MP3, as well as spliceosome, termination of polymerase II transcription regulation of stem cell population maintenance, histone demethylase activity, response to vitamin D, regulation of DNA methylation, snRNA processing, negative regulation of cell proliferation, regulation of cellular senescence, protein import into mitochondrial matrix, catabolism of misfolded protein, nonsense mediated mRNA decay, mitochondrial morphogenesis and transport, and cell differentiation.

MP2 was further characterised by stronger signals detected in apoptotic and autophagy pathways compared to MP1 or MP3. In particular, signalling by p53 class mediators showed increased predictivity in this subtype. This may indicate a higher efficiency in clearing cells with unrepairable DNA damage from the tissue.

MP2 and MP3 further showed higher activities in pigment metabolic processes, which is in concordance with the stronger tanning responses observed in more UV tolerant skin.

MP3 was characterised by the strongest pathway signals detected in cell cycle checkpoint and DNA synthesis pathways, as well as genes involved with chromosome condensation. These findings may indicate a higher sensitivity of the DNA damage sensing machinery in MP3 subjects in response to irradiation, which may provide a more tightly regulated cessation of DNA replication and thus more time for the repair of UV induced DNA damage. Further, MP3 was characterized by a stronger oxidoreduction coenzyme metabolic process response, tRNA modification, reverse cholesterol transport, nucleotide salvage, water homeostasis, DNA packaging, histone phosphorylation, and maintenance of epithelial cell polarity.

In summary, the analyses reveal previously unknown divergent molecular responses to UV irradiation in the identified Molecular Phototypes. This high-resolution *in vivo* data may help explain inter-individual UV tolerance, and provides evidence of a melanin-independent form of UV-induced damage protection in the skin of subjects with higher innate UV resilience.

**Claims**

1. A method for classifying humans into Molecular Phototypes based on the biological response of their skin to UV irradiation, the method comprising:

   a) providing skin cells irradiated with UV light obtained from a plurality of human subjects;
   b) determining the methylation levels and/or expression levels of at least about 100 features in said skin cells, wherein the features are selected from CpG sites and RNAs;
   c) using the methylation data and/or expression data obtained in step b) to generate a subject similarity network;
   d) performing cluster analysis on the subject similarity network; and
   e) defining Molecular Phototypes based on the clusters in the subject similarity network.

2. The method of claim 1, wherein step b) comprises determining the methylation levels of:

   i) at least about 2,500, at least about 5,000, at least about 10,000, at least about 20,000, or at least about 40,000 CpG sites; or
   ii) at least about 0.3%, at least about 0.6%, at least about 1.2%, at least about 2.5%, or at least about 5% of the CpG sites in the Infinium MethylationEPIC BeadChip array.

3. The method of claim 1 or 2, wherein step b) comprises determining the expression levels of:

   i) at least about 100, at least about 200, at least about 400, at least about 800, or at least about 1,600 RNAs; or

ii) at least about 0.3%, at least about 0.6%, at least about 1.2%, at least about 2.5%, or at least about 5% of the RNAs in the transcriptome of the skin cells.

4. The method of any preceding claim, wherein step b) comprises determining the methylation level of at least one CpG site and determining the expression level of at least one RNA.

5. The method of claim 4, wherein step c) comprises:

   i) generating a subject similarity network for the CpG sites methylation data obtained in step b);
   ii) generating a subject similarity network for the RNA expression data obtained in step b); and
   iii) integrating the two subject similarity networks into a single, fused similarity network.

6. The method of any preceding claim, wherein step b) comprises determining the methylation levels of:

   i) at least about 2,500, at least about 5,000, at least about 10,000, at least about 20,000, or at least about 40,000 CpG sites, or
   ii) at least about 0.3%, at least about 0.6%, at least about 1.2%, at least about 2.5%, or at least about 5% of the CpG sites in the Infinium MethylationEPIC BeadChip array,

   and the expression levels of:

   iii) at least about 100, at least about 200, at least about 400, at least about 800, , or at least about 1,600 RNAs, or
   iv) at least about 0.3%, at least about 0.6%, at least about 1.2%, at least about 2.5, or at least about 5% of the RNAs in the transcriptome of the skin cells.

7. The method of any preceding claim, wherein the CpG sites' methylation levels and/or the RNAs' expression levels correlate with MED.

8. The method of any preceding claim, wherein the CpG sites fall in the 1%, 5%, 10%, or 20% of CpG sites in the Infinium MethylationEPIC BeadChip array whose methylation levels correlate most strongly with MED and/or wherein the RNAs falls in the 1%, 5%, 10%, or 20% of RNAs in the transcriptome whose expression levels correlate most strongly with MED.

9. The method of any preceding claim, wherein the cluster analysis is performed using spectral clustering, Hierarchical clustering, k-means, k-medoids, expectation-maximization, affinity propagation, density-based clustering, self-organizing maps, or community detection algorithms, optionally louvain clustering.

10. The method of any preceding claim, wherein the subject similarity network identifies three distinct clusters, which are designated as three Molecular Phototypes.

11. The method of claim 10, wherein:

   i) a first cluster is **characterised by** differential regulation of biological pathways relating to inflammasome activation, interleukin and general cytokine response, protein modification, unfolded protein response, lipid biosynthetic and catabolic processes, regulation of cell adhesion, steroid hormone receptor activity, necrotic cell death, macrophage activation, activation of positive chemotaxis, phagocytosis, and/or response to pain;
   ii) a second cluster is **characterised by** differential regulation of biological pathways relating to type I interferon response, spliceosome, termination of polymerase II transcription regulation of stem cell population maintenance, histone demethylase activity, response to vitamin D, regulation of DNA methylation, snRNA processing, negative regulation of cell proliferation, regulation of cellular senescence, protein import into mitochondrial matrix, catabolism of misfolded protein, nonsense mediated mRNA decay, mitochondrial morphogenesis and transport, and/or cell differentiation; and/or
   iii) a third cluster is **characterised by** differential regulation of biological pathways relating to cell cycle checkpoints, DNA synthesis, chromosome condensation, oxidoreduction coenzyme metabolic process response, tRNA modification, reverse cholesterol transport, nucleotide salvage, water homeostasis, DNA packaging, histone phosphorylation, and/or maintenance of epithelial cell polarity.

12. A computer program for classifying humans into Molecular Phototypes based on the biological response of their skin to UV irradiation, the computer program comprising instructions which, when the program is executed by a computer, cause the computer to carry out the following steps:

   a) inputting the methylation levels and/or expression levels of at least about 100 features in skin cells irradiated with UV light obtained from a plurality of human subjects, wherein the features are selected from CpG sites and RNAs;
   b) using the methylation data and/or expression data obtained in step b) to generate a subject similarity network;

c) performing cluster analysis on the subject similarity network; and

d) outputting definitions of Molecular Phototypes based on the clusters in the subject similarity network.

13. A method for identifying biological pathways that are associated with the response to UV irradiation of skin in human subjects belonging to a Molecular Phototype determined by the method of any one of claims 1 to 11 or the computer program of claim 12, the method comprising:

a) providing skin cells irradiated with UV light and control skin cells obtained from a plurality of human subjects belonging to a Molecular Phototype determined by the method of any of claims 1-11 or the computer program of claim 12;

b) determining the expression levels of at least one gene in said irradiated and control skin cells, wherein said at least one gene is annotated to a biological pathway;

c) using a machine-learning model trained on the data obtained in step b) to identify whether said at least one gene can discriminate between irradiated and control skin cells; and

d) determining the strength of the association between the biological pathway and the response to UV irradiation of skin in the human subjects belonging to the Molecular Phototype, wherein the strength of the association is based on the degree to which the machine-learning model can discriminate between irradiated and control skin cells.

14. The method of claim 13, wherein the at least one gene is a set of genes annotated to a biological pathway.

15. The method of claim 14, wherein the set of genes annotated to a biological pathway is selected from the sets of genes in:

i) the GO term collection;
ii) the WikiPathways collection;
iii) the KEGG pathway collection;
iv) the BioCarta pathway collection;
v) the Reactome pathway collection; and/or
vi) the Hallmark gene set collection.

16. The method of any of claims 13-15, wherein the biological pathway is identified as being associated with the skin's response to UV irradiation in human subjects belonging to the Molecular Phototype if the at least one gene is able to discriminate between irradiated and control skin cells with at least about 70, at least about 80, at least about 90, or at least about 95% accuracy.

17. The method of any of claims 13-16, wherein the machine learning model has been trained on data comprising irradiation statuses and expression levels of the at least one gene.

18. The method of any of claims 13-17, wherein the machine learning model is a support vector machine (SVM), a logistic regression model, a decision tree, a random forest, gradient boosting, or an artificial neural network.

19. The method of any of claims 1-11 or 13-18, wherein the skin cells comprise as a majority, consist essentially of, or consist of epidermis cells and/or dermis cells.

20. The method of any of claims 1-11 or 13-19, wherein:

i) the UV irradiated skin cells have been irradiated with an amount of UV light that achieved an MED of from about 0.5 to about 1.3, from about 0.7 to about 1.1, from about 0.8 to about 1.0, or about 0.9, and/or

ii) the UV irradiated skin cells have been irradiated at least 2 times, at least 3 times, from 2 to 4 times, or 3 times, optionally wherein the irradiations have been performed 12 hours to about 48 hours apart, about 12 hours to about 36 hours apart, about 20 to about 28 hours apart, or about 24 hours apart.

21. A computer program for identifying biological pathways that are associated with the response to UV irradiation of skin in human subjects belonging to a Molecular Phototype as determined by the method of any of claims 1-11 or the computer program of claim 12, the computer program comprising instructions which, when the program is executed by a computer, cause the computer to carry out the following steps:

a) inputting the expression level of at least one gene annotated to a biological pathway obtained from skin cells irradiated with UV light and control skin cells obtained from a plurality of human subjects belonging to a Molecular Phototype as determined by the method of any of claims 1-11 or the computer program of claim 12;

b) using a machine-learning model trained on the data obtained in step a) to identify whether said at least one gene can discriminate between irradiated and control skin cells;

c) determining the strength of the association between the biological pathway and the response to UV irradiation of skin in the human subjects belonging to the Molecular Phototype, wherein the strength of the association is based on the degree to which the machine-learning model can discriminate between irradiated and control skin cells; and

d) outputting a decision on whether the biological pathway is associated with the response to UV irradiation of skin in human subjects belonging to the Molecular Phototype.

Figure 1

Figure 2

**EP 3 854 881 A1**

Europäisches Patentamt
European Patent Office
Office européen des brevets

# EUROPEAN SEARCH REPORT

Application Number

EP 20 15 3236

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 372 481 A2 (VILLA BORGHINI S R L [IT]) 2 January 2004 (2004-01-02) * Claim 1, [0029] * | 1-21 | INV. C12Q1/68 C12Q1/6876 |
| Y | WO 2019/238792 A1 (UNILEVER PLC [GB]; UNILEVER NV [NL]; CONOPCO INC D/B/A UNILEVER [US]) 19 December 2019 (2019-12-19) * (p35, lines 19-28), (p36, lines 1-8), Firgure 3 * | 1,2 | |
| Y | WO 2011/109224 A1 (US HEALTH [US]; TERUNUMA ATSUSHI [US]; FALLOON ELIZABETH [US]) 9 September 2011 (2011-09-09) * claims; examples * | 3,7,8 | |
| Y | US 2019/078162 A1 (REIS DE OLIVEIRA CAROLINA [US] ET AL) 14 March 2019 (2019-03-14) * claims * | 1-21 | |
| A | US 2011/045471 A1 (PARR RYAN [CA] ET AL) 24 February 2011 (2011-02-24) * [0099], [0092] * | 3-21 | **TECHNICAL FIELDS SEARCHED (IPC)** C12Q |

The present search report has been drawn up for all claims

| Place of search | Date of completion of the search | Examiner |
|---|---|---|
| Munich | 5 June 2020 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

22

## ANNEX TO THE EUROPEAN SEARCH REPORT
## ON EUROPEAN PATENT APPLICATION NO.

EP 20 15 3236

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2020

| Patent document cited in search report | | | Publication date | Patent family member(s) | | | Publication date |
|---|---|---|---|---|---|---|---|
| EP | 1372481 | A2 | 02-01-2004 | AT | 307529 | T | 15-11-2005 |
| | | | | AU | 2002253537 | B2 | 24-11-2005 |
| | | | | DE | 60206892 | T2 | 27-07-2006 |
| | | | | EP | 1372481 | A2 | 02-01-2004 |
| | | | | IT | FI20010055 | A1 | 30-09-2002 |
| | | | | US | 2004097812 | A1 | 20-05-2004 |
| | | | | WO | 02078543 | A2 | 10-10-2002 |
| WO | 2019238792 | A1 | 19-12-2019 | NONE | | | |
| WO | 2011109224 | A1 | 09-09-2011 | AU | 2011223937 | A1 | 06-09-2012 |
| | | | | CA | 2791662 | A1 | 09-09-2011 |
| | | | | EP | 2542691 | A1 | 09-01-2013 |
| | | | | US | 2013065781 | A1 | 14-03-2013 |
| | | | | WO | 2011109224 | A1 | 09-09-2011 |
| US | 2019078162 | A1 | 14-03-2019 | US | 2019078162 | A1 | 14-03-2019 |
| | | | | WO | 2019074615 | A2 | 18-04-2019 |
| US | 2011045471 | A1 | 24-02-2011 | AU | 2008310257 | A1 | 16-04-2009 |
| | | | | CN | 101896622 | A | 24-11-2010 |
| | | | | EP | 2217730 | A1 | 18-08-2010 |
| | | | | ES | 2523684 | T3 | 28-11-2014 |
| | | | | HK | 1145408 | A1 | 31-07-2015 |
| | | | | JP | 5646998 | B2 | 24-12-2014 |
| | | | | JP | 2011500004 | A | 06-01-2011 |
| | | | | KR | 20100089070 | A | 11-08-2010 |
| | | | | NZ | 584595 | A | 21-12-2012 |
| | | | | US | 2011045471 | A1 | 24-02-2011 |
| | | | | WO | 2009046535 | A1 | 16-04-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **FITZPATRICK.** Soleil et peau. *J Med Esthet,* 1975, vol. 2, 33-34
- **EILERS et al.** Accuracy of Self-report in Assessing Fitzpatrick Skin Phototypes I through VI. *JAMA Dermatol.,* 2013, vol. 149 (11), 1289-1294
- **PAI ; BADER.** Patient Similarity Networks for Precision Medicine. *J Mol Biol.,* 2018, vol. 430, 2924-2938
- **WANG et al.** Similarity network fusion for aggregating data types on a genomic scale. *Nat Methods,* 2014, vol. 11 (3), 333-7
- **LIBERZON et al.** Molecular signatures database (MSigDB) 3.0. *Bioinformatics,* 2011, vol. 27, 1739-1740, http://software.broadinstitute.org/gsea/msigdb/genesets.jsp
- **HECKMAN et al.** Minimal Erythema Dose (MED) Testing. *J Vis Exp.,* 2013, vol. 75, 50175
- **SÜDEL et al.** Tight control of matrix metalloproteinase-1 activity in human skin. *Photochem Photobiol.,* 2003, vol. 78, 355-60
- **LOVE et al.** Moderated estimation of fold change and dispersion for RNA-seq data with DESeq2. *Genome Biol.,* 2014, vol. 15, 550
- **ARYEE.** Minfi: a flexible and comprehensive Bioconductor package for the analysis of Infinium DNA methylation microarrays. *Bioinformatics,* 2014, vol. 30, 1363-1369
- **LEE et al.** Inferring Pathway Activity toward Precise Disease Classification. *PLoS Comput Biol.,* 2008, vol. 4, e1000217
- **HÄNZELMANN et al.** GSVA: gene set variation analysis for microarray and RNA-seq data. *BMC Bioinformatics,* 2013, vol. 14, 7
- **LIBERZON et al.** The Molecular Signatures Database Hallmark Gene Set Collection. *Cell Syst.,* 2015, vol. 1, 417-425
- **LIBERZON et al.** Molecular signatures database (MSigDB) 3.0. *Bioinformatics,* 2011, vol. 27, 1739-1740
- **WANG et al.** Similarity network fusion for aggregating data types on a genomic scale. *Nat Methods,* 2014, vol. 11, 333-337
- **DIMITRIADOU et al.** *Misc Functions of the Department of Statistics (e1071), TU Wien. R package version 1,* 2011, e1071
- **BISCHL et al.** mlr: Machine Learning in R. *J Mach Learn Res.,* 2016, vol. 17, 1-5